# EUROPÄISCHE PATENTSCHRIFT

(11) **EP 0 596 177 B1**
(45) Veröffentlichungstag und Bekanntmachung des Hinweises auf die Patenterteilung: **07.01.1998**
(21) Anmeldenummer: 92810839.8
(22) Anmeldetag: 02.11.1992
(51) Int. Cl.: A61F 2/08

(54) **Verankerung für ein künstliches Band, insbesondere ein Kreuzband eines Kniegelenks**
Anchor for a synthetic ligament, in particular for a cruciate knee-ligament
Ancrage pour un ligament synthétique, en particulier pour un ligament croisé du genou

(43) Veröffentlichungstag der Anmeldung: 11.05.1994
(73) Patentinhaber: Sulzer Orthopädie AG, 6340 Baar (CH)
(72) Erfinder: Zimmermann, Martin, CH-3645 Gwatt/Thun (CH); Tendon, Joel, CH-2520 La Neuveville (CH)
(74) Vertreter: Trieblnig, Adolf

(56) Entgegenhaltungen:
- EP-A- 0 238 223
- EP-A- 0 330 328

## Beschreibung

Die Erfindung betrifft eine Verankerung zum Befestigen eines künstlichen Bandes entsprechend dem Oberbegriff des Patentanspruchs 1.

Eine Anordnung mit Verankerungen der eingangs genannten Art ist aus der EP-A-0 330 328 bekannt. Die bekannten Verankerungen enthalten je ein hülsenförmiges Halteelement für ein in einem der Gelenkteile eines Kniegelenks zu befestigendes künstliches Kreuzband, welches durch zwei je in einem der Gelenkteile ausgebildete Führungskanäle hindurchgeführt und beidenends je mit einem der Halteelemente verbunden ist. Die Halteelemente enthalten je eine in den Führungskanal einsetzbare Hülse mit zwei in deren Längsrichtung, jedoch nicht koaxial angeordneten Bohrungen, welche über eine abgestufte Schulter ineinander übergehen, und eine quer zu den Längsbohrungen angeordnete dritte Bohrung, deren Achse die abgestufte Schulter unter einem Winkel von 90° schneidet. Die dritte Bohrung ist zur Aufnahme einer Klemmschraube bestimmt, durch welche der betreffende Endabschnitt des Bandes an die abgestufte Schulter angepresst und unter Vorspannung gehalten werden kann.

Bei der bekannten Ausführung werden die durch das Band eingeleiteten Zugkräfte über die im Führungskanal in Zugrichtung abgestützte Hülse auf das Knochengewebe des betreffenden Gelenkteils übertragen.

Eine andere, aus der EP-A-0 465 408 bekannte Verankerung enthält eine in den Führungskanal einsetzbare konische Verankerungshülse als Halteelement und eine in diese selbsthemmend einsetzbare, radial deformierbare Klemmhülse zum Festhalten des Bandes. Eine weitere, aus der EP-A-0 232 049 bekannte Verankerung enthält konische Klemmelemente, die jeweils in einen strumpfartigen Bandfortsatz eingeschoben und über diesen gegen eine in den Führungskanal einsetzbare Verankerungshülse verspannt werden, welche ihrerseits nachgespannt werden kann. Die beiden zuletzt genannten Verankerungen erfordern zusätzliche Einrichtungen und/oder relativ aufwendige Einstellarbeiten zum Spannen oder gegebenenfalls zum Nachspannen des implantierten Bandes, um die gegenläufigen Bewegungen von Bandstreckung und Klemmen so zu koordinieren, dass sich das Band mit der gewollten Vorspannung festsetzt.

Der Erfindung liegt die Aufgabe zugrunde, eine Verankerung der eingangs genannten Art in einer einfachen Bauweise zu schaffen, welche mit geringem Arbeits- und Zeitaufwand eine genaue, bleibende Positionierung des Halteelements ermöglicht und zugleich eine genaue Einstellung und/oder Nachstellung einer vorbestimmten Vorspannung des implantierten Bandes gewährleistet.

Diese Aufgabe wird gemäss der Erfindung durch die im kennzeichnenden Teil des Patentanspruchs 1 angegebenen Merkmale gelöst.

Die erfindungsgemäss ausgebildete Verankerung ermöglicht eine das Knochengewebe schonende Anbringung des Halteelements und gestattet eine sichere Befestigung des implantierten Bandes sowie eine funktionelle Trennung der für die Einstellung der Vorspannung des Bandes und der für die Einstellung der Klemmkraft erforderlichen Arbeitsschritte, indem die Klemmkraft unabhängig von der jeweiligen Vorspannung des Bandes von aussen einstellbar ist. Durch die erfindungsgemässe Anordnung ergibt sich ferner eine einfache Vorbearbeitung im Knochen, indem der zur Aufnahme des Bandes bestimmte Führungskanal und eine ihn überschneidende, zur Aufnahme des Stiftes bestimmte Bohrung je ausschliesslich auf die Abmessungen des betreffenden Teils - des Bandes bzw. des Stiftes - abgestimmt und ohne weitere Nachbearbeitungen ausgeführt werden können. Durch den quer zur Zugrichtung angeordneten Stift können zudem die über das Band eingeleiteten Zugkräfte ausserhalb des Führungskanals, und damit im wesentlichen ohne Belastung der relativ weichen inneren Partie des Knochengewebes auf den Knochen übertragen werden. Bei der erfindungsgemässen Ausführung kann das Band auf einfache Weise durch den Führungskanal und die Durchtrittsöffnung des Stiftes hindurch in den Knochen eingezogen und an der Aussenseite des Knochens, z.B. mit einer Federwaage, unter Vorspannung gehalten werden, während die Klemmung des Bandes davon unabhängig durch das Klemmelement erfolgt, welches durch die axiale Bohrung des Stiftes gegen den die Durchtrittsöffnung durchsetzenden Abschnitt des Bandes geführt und über diesen gegen eine Stützfläche des Stiftes verspannt wird. Das Band kann von einem Endlosstapel in der passenden Länge eingezogen und nach dem Klemmen bündig zur Aussenfläche des Knochens abgeschnitten werden. Auch die Teile der Verankerung können im wesentlichen bündig mit der Knochenoberfläche ausgeführt werden.

Ausgestaltungen der Erfindung sind in den abhängigen Ansprüchen angegeben.

Weitere Einzelheiten ergeben sich aus der folgenden Beschreibung von in der Zeichnung schematisch dargestellten Ausführungsbeispielen der Erfindung, in Verbindung mit den Patentansprüchen. In der Zeichnung zeigen:
- Fig. 1: einen mit einer erfindungsgemäss ausgebildeten Verankerung versehenen Teil eines Knochens in einem Längsschnitt;
- Fig. 2: die Verankerung nach Fig. 1 in einer grösseren Schnittdarstellung und
- Fig. 3: eine entsprechende Verankerung nach einer abgewandelten Ausführungsform der Erfindung.

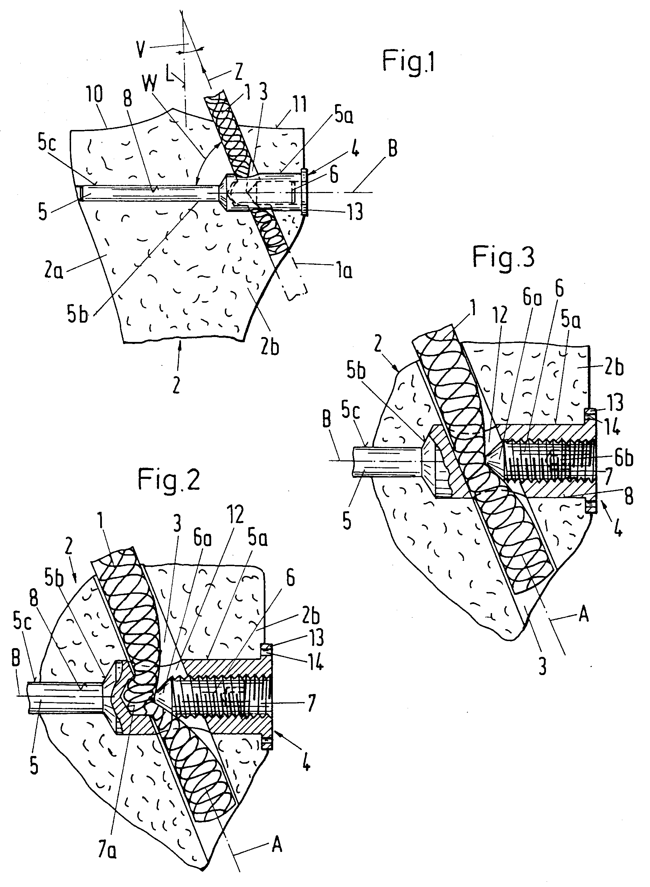

Gemäss Fig. 1 ist ein Ende eines künstliches Bandes 1, beim dargestellten Beispiel eines künstlichen Kreuzbandes eines Kniegelenks, durch einen in einem Knochen 2, darstellungsgemäss im Schienbein (Tibia), ausgebildeten Führungskanal 3 hindurchgeführt und in einer Verankerung 4 gehalten. Das andere Ende des Bandes 1 ist in einem nicht dargestellten Gegenstück zum Knochen 1, beim vorliegenden Beispiel im Oberschenkelknochen (Femur), gehalten, welcher mit einer entsprechenden Verankerung 4 versehen sein kann.

Der Führungskanal 3 ist durch eine Bohrung gebildet, die den Knochen 2 unter einem spitzen Winkel V zu seiner Längsachse L durchsetzt. Die Verankerung 4 enthält ein Halteelement in Form eines quer zur Zugrichtung (Pfeil Z) des Bandes 1 einsetzbaren Stiftes 5 und ein Klemmelement in Form einer stellschraube 6, die in einer axialen Bohrung 7 des Stiftes 5 angeordnet ist und die darstellungsgemäss mit einem spitzen Ende 6a sowie mit einem Innensechskant 6b ausgeführt sein kann. Der Stift 5 ist in einer den Knochen 2 durchquerenden, abgesetzten Bohrung 8 angeordnet, welche den Führungskanal 3 überschneidet und welche mit dem in Zugrichtung (Pfeil Z) des Bandes 1 gegen das Innere des Knochens 2 verlaufenden Abschnitt des Führungskanals 3 einen Winkel W einschliesst, der kleiner ist als 90°. Entsprechend wird auf den Stift 5 eine durch den Winkel W bestimmte, in axialer Richtung wirkende Komponente der jeweils über das Band 1 eingeleiteten Zugkraft ausgeübt, durch die der Stift 5 über eine an ihm ausgebildete Kragenpartie 13 in Fig. 1 nach links gegen das Innere des Knochens 2 verspannbar ist. Beim dargestellten Beispiel verläuft die Bohrung 8 annähernd parallel zu dem durch Kondylen 10 und 11 des Knochens 2 bestimmten "Tibiaplateau", wobei der Winkel W ca. 60° bis 70° beträgt. Es vesteht sich, dass jeder beliebige Winkel W gewählt werden kann, der eine als ausreichend erachtete Komponente der Zugkraft gewährleistet.

Die Stellschraube 6 ist in einem Endabschnitt 5a des Stiftes 5 angeordnet, der über eine kegelige Absatzpartie 5b in einen restlichen Längenabschnitt 5c kleineren Durchmessers übergeht. Der Endabschnitt 5a ist mit einer ihn in Querrichtung durchsetzenden, zum Hindurchführen des Bandes 1 geeigneten Durchtrittsöffnung 12 ausgeführt, die beim dargestellten Beispiel durch eine mit dem Führungskanal 3 fluchtend positionierbare Bohrung gebildet ist, deren Durchmesser demjenigen des Führungskanals 3 entspricht und deren Achse A mit der Längsachse B des Stiftes 5 einen Winkel einschliesst, der dem Winkel W zwischen dem Führungskanal 3 und der Bohrung 8 des Knochens 2 entspricht. Anstelle der dargestellten schrägen Bohrung kann auch ein entsprechender, in Querrichtung durchgehender Schlitz als Durchtrittsöffnung 12 vorgesehen sein. In die Durchtrittsöffnung 12 mündet die mit einem Innengewinde versehene axiale Bohrung 7 des Stiftes 5, in der die Stellschraube 6 - beim dargestellten Beispiel mit einem Sechskantschlüssel - verstellbar und gegen den die Durchtrittsöffnung 12 durchsetzenden Abschnitt des Bandes 1 verspannbar gehalten ist. Entsprechend der Darstellung nach den Fig. 1 und 2 kann die axiale Bohrung 7 mit einer Tiefe ausgeführt sein, die sich über das entsprechende Mass der Durchtrittsöffnung 12 hinaus erstreckt, so dass das Band 1 beim Anziehen der Stellschraube 6 in den Endabschnitt 7a der Bohrung 7 hineingedrängt und damit eine zusätzliche Sicherung der Bandbefestigung erreicht wird.

Entsprechend der Darstellung nach Fig. 3 kann die axiale Bohrung 7 des Stiftes 5 auch in der Durchtrittsöffnung 12 enden, so dass das Band 1 durch die Stellschraube 6 - oder ein entsprechendes anderes Klemmelement - an die Wandung der Durchtrittsöffnung 12 angepresst und ohne zusätzliche Verformung entsprechend schonender gehalten werden kann.

Der Stift 5 kann vorzugsweise als über die betreffende Querschnittsabmessung des Knochens 2 durchgehender Bauteil ausgeführt sein, der sich über die beiden im Querschnitt einander gegenüberliegenden Randpartien 2a und 2b der Kortikalis des Knochens 2 erstreckt und sich mit seinen beiden Enden in diesen relativ harten Randpartien 2a und 2b des Knochengewebes abstützt. Entsprechend wird eine Beanspruchung der relativ weichen inneren Partie, der Spongiosa, des Knochengewebes durch den Stift 5 vermieden und eine bleibende, genaue Positionierung des Stiftes 5 gewährleistet.

Wie aus der Zeichnung hervorgeht, kann die Kragenpartie 13 durch einen am Endabschnitt 5a vorgesehenen flanschartigen Ansatz gebildet sein, über den sich der Stift 5 an der Aussenseite des Knochens 2 abstützt. Die Kragenpartie 13 kann im wesentlichen bündig mit der Aussenseite des Knochens 2 abschliessen oder über diese, wie dargestellt, um ein geringes Mass, z.B. 1 mm, vorstehen. Darstellungsgemäss kann ferner die Kragenpartie 13 mit Bohrungen 14 zur Aufnahme von Mitnehmerzapfen eines nicht dargestellten Setzinstrumentes versehen sein, durch welches der Stift 5 eingesetzt, verstellt und entfernt werden kann.

Zum Implantieren des Bandes 1 in den Knochen 2 wird z.B. in einem ersten Schritt eine einer vorbestimmten Lage des Führungskanals 3 entsprechende erste Bohrung in das Knochengewebe eingebracht. Hierauf wird in einem zweiten Schritt, z. B. mittels einer nicht dargestellten Bohrlehre bekannter Art, unter dem vorbestimmten Winkel W eine die erste Bohrung überschneidende, zur Aufnahme des Längenabschnitts 5c des Stiftes 5 bestimmte, durchgehende zweite Bohrung 8 in den Knochen eingebracht, die im Ueberschneidungsbereich entsprechend den Abmessungen des aufzunehmenden Endabschnitts 5a des Stiftes 5 erweitert wird. In einem dritten Schritt wird der mit der Durchtrittsöffnung 12 versehene Stift 5 in die Bohrung 8 eingeführt und mit zum Führungskanal 3 fluchtender Durchtrittsöffnung 12 positioniert, wobei die Absatzpartie 5b an eine entsprechende Schulterpartie der Bohrung 8 angelegt werden kann. In einem vierten Schritt wird das künstliche Band 1 durch den durch den Führungskanal 3 und die Durchtrittsöffnung 12 gebildeten Durchgang hindurchgeführt, wobei das in Fig. 1 strichpunktiert dargestellte untere Bandende 1a ausserhalb des Führungskanals 3 erfasst und gegebenenfalls, etwa mittels einer Federwaage, auf eine vorbestimmte Vorspannung gebracht werden kann, wenn das andere Bandende bereits am nicht dargestellten Gegenstück des Gelenks verankert ist. In einem fünften Schritt wird schliesslich die Stellschraube 6 in die Bohrung 7 des Stiftes 5 eingeführt und über den die Durchtrittsöffnung 12 durchsetzenden Abschnitt des Bandes 1 gegen eine dem Band 1 zugekehrte Stützpartie des Stiftes 5 verspannt, worauf das aus dem Führungskanal 3 vorstehende Bandende 1a zur Aussenfläche des Knochens 2 bündig abgeschnitten wird.

Es versteht sich, dass auch die zur Aufnahme des Stiftes 5 bestimmte Bohrung 8 als erste Bohrung und der Führungskanal 3 als zweite Bohrung ausgeführt werden kann. Anstelle der dargestellten Stellschraube können beliebig andere Schraubentypen oder ein entsprechendes, z.B. federnd einsetzbares Klemmelement, verwendet werden. Die erfindungsgemässe Verankerung ist auch für andere Anwendungen, z.B. im Bereich der Wirbelsäule oder des Schultergelenks, geeignet.

## Patentansprüche

1. Verankerung zum Befestigen eines künstlichen Bandes (1), insbesondere eines Kreuzbandes für ein Kniegelenk, in einem Knochen (2), wobei die Verankerung ein im Knochen (2) anbringbares Halteelement zur Aufnahme eines dieses in einer Zugrichtung (Z) durchsetzenden Endabschnitts des Bandes (1) und ein gegen diesen Endabschnitt verspannbares, am Halteelement quer zur Zugrichtung (Z) beweglich anbringbares Klemmelement enthält, dadurch gekennzeichnet, dass als Halteelement ein quer zur Zugrichtung (Z) des Bandes (1) einsetzbarer Stift (5) vorgesehen ist, welcher eine ihn quer zu seiner Längsachse (B) durchsetzende Durchtrittsöffnung (12) zum Hindurchführen des Endabschnitts des Bandes (1) und eine in diese Durchtrittsöffnung (12) mündende axiale Bohrung (7) enthält, in der das Klemmelement in Richtung der Längsachse (B) des Stiftes (5) einstellbar gehalten ist, wobei diese Längsachse (B) mit der Längsachse der Durchtrittsöffnung (12) des Bandes (1) einen Winkel (W) einschliesst, der kleiner ist als 90°.

2. Verankerung nach Anspruch 1, dadurch gekennzeichnet, dass der Stift (5) als über eine Querschnittsabmessung einer zu seiner Aufnahme bestimmten Knochenpartie durchgehend positionierbarer Bauteil ausgebildet ist, der dazu eingerichtet ist, sich mit seinen Enden in zwei einander gegenüberliegenden Randpartien (2a, 2b) der Knochenpartie abzustützen.

3. Verankerung nach Anspruch 1 oder 2, dadurch gekennzeichnet, dass die Durchtrittsöffnung (12) des Stiftes (5) durch eine Bohrung gebildet ist.

4. Verankerung nach einem der vorangehenden Ansprüche, dadurch gekennzeichnet, dass die Durchtrittsöffnung (12) und die axiale Bohrung (7) an einem Endabschnitt (5a) des Stiftes (5) ausgebildet sind, der mit einem grösseren Durchmesser ausgeführt ist als der an ihn anschliessende restliche Längenabschnitt (5c) des Stiftes (5).

5. Verankerung nach Anspruch 4, dadurch gekennzeichnet, dass der Endabschnitt (5a) und der restliche Längenabschnitt (5c) des Stiftes (5) über eine an eine Schulterpartie einer Bohrung (8) anlegbare kegelige Absatzpartie (5b) miteinander verbunden sind.

6. Verankerung nach einem der vorangehenden Ansprüche, dadurch gekennzeichnet, dass der Stift (5) mit einer an die Aussenseite des Knochens (2) anlegbaren, flanschartig abstehenden Kragenpartie (13) ausgeführt ist.

7. Verankerung nach einem der vorangehenden Ansprüche, dadurch gekennzeichnet, dass die axiale Bohrung (7) des Stiftes (5) als Gewindebohrung ausgeführt ist, und dass das Klemmelement als in dieser verstellbare Stellschraube (6) ausgeführt ist.

8. Verankerung nach einem der vorangehenden Ansprüche, dadurch gekennzeichnet, dass die axiale Bohrung (7) des Stiftes (5) mit einer Tiefe ausgeführt ist, die sich über die Durchtrittsöffnung (12) hinaus erstreckt.

## Claims

1. An attachment for fixing an artificial ligament (1), in particular a cruciate ligament for a knee joint, in a bone (2), with the attachment containing a retaining element, which can be fitted in the bone (2), for receiving an end section of the ligament (1) penetrating said element in a pulling direction (Z) and a clamping element which can be braced against this end section and can be movably fitted on the retaining element at right angles to the pulling direction (Z),
**characterised in that** as the retaining element there is provided a pin (5) insertable at right angles to the pulling direction (Z) of the ligament (1), which pin contains a through-hole (12) traversing it at right angles to its longitudinal axis (B) for passing through the end section of the ligament (1) and an axial bore hole (7) opening into this through-hole (12), in which the clamping element is adjustably retained in the direction of the longitudinal axis (B) of the pin (5), with this longitudinal axis (B) with the longitudinal axis of the through-hole (12) of the ligament (1) enclosing an angle (W) which is less than 90°.

2. An attachment according to Claim 1,
**characterised in that** the pin (5) is constructed as a component which can be positioned passing through a cross-sectional dimension of a bone part intended to receive it, which is fitted to be supported by its ends in two end parts (2a, 2b) of the bone part opposite one another.

3. An attachment according to Claim 1 or 2,
**characterised in that** the through-hole (12) of the pin (5) is formed by a bore hole.

4. An attachment according to one of the preceding Claims,
**characterised in that** the through-hole (12) and the axial bore hole (7) are constructed at an end section (5a) of the pin (5), which is designed with a larger diameter than the remaining oblong section (5c) of the pin (5) connected thereto.

5. An attachment according to Claim 4,
**characterised in that** the end section (5a) and the remaining oblong section (5c) of the pin (5) are connected to one another via a tapered neck part (5b) which can be positioned on a shoulder part of a bore hole (8).

6. An attachment according to one of the preceding Claims,
**characterised in that** the pin (5) is designed with a collar part (13) which can be positioned on the outside of the bone (2) and protrudes like a flange.

7. An attachment according to one of the preceding Claims,
**characterised in that** the axial bore hole (7) of the pin (5) is constructed as a taphole,
**and in that** the clamping element is constructed as a locking screw (6) adjustable therein.

8. An attachment according to one of the preceding Claims,
**characterised in that** the axial bore hole (7) of the pin (5) is constructed with a depth which extends beyond the through-hole (12).

## Revendications

1. Ancrage pour la fixation d'un ligament artificiel (1) notamment d'un ligament croisé pour une articulation du genou, dans un os (2), l'ancrage comportant un élément de retenue pouvant être disposé dans l'os (2) pour recevoir un tronçon d'extrémité du ligament (1) traversant celui-ci dans une direction de traction (Z) et un élément de serrage pouvant être tendu contre ce tronçon d'extrémité, pouvant être monté de façon mobile sur l'élément de retenue transversalement à la direction de traction (Z), caractérisé en ce qu'il est prévu comme élément de retenue une tige (5) pouvant être insérée transversalement à la direction de traction (Z) de la bande (1) qui présente une ouverture de passage (12) passant à travers celle-ci transversalement à son axe longitudinal (B) en vue du passage du tronçon d'extrémité du ligament (1) ainsi qu'un perçage axial (7) débouchant dans cette ouverture de passage (12) dans lequel l'élément de serrage est retenu de manière réglable en direction de l'axe longitudinal (B) de la tige (5), cet axe longitudinal (B) formant avec l'axe longitudinal de l'ouverture de passage (12) de la bande (1) un angle (W) qui est plus petit que 90°.

2. Ancrage selon la revendication 1, caractérisé en ce que la tige (5) est réalisée en tant que composant apte à être positionné d'une manière traversante sur une dimension en section transversale d'une partie osseuse prévue pour sa réception et qui est agencée pour prendre appui avec ses extrémités dans deux parties de bord opposées (2a, 2b) de la partie osseuse.

3. Ancrage selon la revendication 1 ou 2, caractérisé en ce que l'ouverture de passage (12) de la tige (5) est formée par un perçage.

4. Ancrage selon l'une des revendications précédentes, caractérisé en ce que l'ouverture de passage (12) et le perçage axial (7) sont réalisés à un tronçon d'extrémité (5a) de la tige (5) qui a un plus grand diamètre que le tronçon longitudinal restant (5c) qui fait suite, de la tige (5).

5. Ancrage selon la revendication 4, caractérisé en ce que le tronçon d'extrémité (5a) et le tronçon longitudinal restant (5c) de la tige (5) sont reliés l'un à l'autre par une partie étagée conique (5b) applicable à une partie d'épaulement d'un perçage (8).

6. Ancrage selon l'une des revendications précédentes, caractérisé en ce que la tige (5) est réalisée avec une partie de col (13) applicable au côté extérieur de l'os (2), faisant saillie en forme de rebord.

7. Ancrage selon l'une des revendications précédentes, caractérisé en ce que le perçage axial (7) de la tige (5) est réalisé sous forme de perçage fileté et en ce que l'élément de serrage est réalisé sous forme de vis de réglage (6) réglable dans celui-ci.

8. Ancrage selon l'une des revendications précédentes, caractérisé en ce que le perçage axial (7) de la tige (5) est réalisé en une profondeur qui va au-delà de l'ouverture de passage (12).
